(19) Europäisches Patentamt

European Patent Office

Office européen des brevets

(11) **EP 1 022 556 A1**

(12) **EUROPÄISCHE PATENTANMELDUNG**

(43) Veröffentlichungstag:
26.07.2000 Patentblatt 2000/30

(51) Int. Cl.[7]: **G01N 11/08**

(21) Anmeldenummer: **99122442.9**

(22) Anmeldetag: **11.11.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **23.12.1998 DE 19859677**

(71) Anmelder:
**GÖTTFERT WERKSTOFF-PRÜFMASCHINEN GMBH
D-74722 Buchen (DE)**

(72) Erfinder:
**Göttfert, Axel, Dr.-Ing.
74722 Buchen (DE)**

(74) Vertreter:
**Naumann, Ulrich, Dr.-Ing.
Patentanwälte,
Ullrich & Naumann,
Luisenstrasse 14
69115 Heidelberg (DE)**

(54) **Vorrichtung zum Bestimmen von rheologischen Eigenschaften einer Schmelze**

(57) Es wird eine Vorrichtung zum Bestimmen von rheologischen Eigenschaften einer Schmelze vorgeschlagen

- mit mindestens einem Schmelzenkanal (2), der endseitig mit einer Meßdüse (3) versehen ist, durch die die Schmelze extrudiert wird,
- mit Mitteln zum Erzeugen und Regeln eines auf die Schmelze wirkenden Gasdrucks und
- mit Mitteln zum Erfassen bzw. Ermitteln des extrudierten Volumenstromes.

Erfindungsgemäß sind mindestens zwei Schmelzenkanäle (2) vorgesehen. Die Schmelzenkanäle (2) sind mit Meßdüsen (3) unterschiedlicher Geometrie ausgestattet. Außerdem besteht eine Druckverbindung zwischen den Schmelzenkanälen (2) in Form von mindestens einer Verbindungsleitung (4), so daß in allen Schmelzenkanälen (2) derselbe Gasdruck auf die Schmelze wirkt.

Fig.

EP 1 022 556 A1

Printed by Xerox (UK) Business Services
2.16.7 (HRS)/3.6

**Beschreibung**

Die Erfindung betrifft eine Vorrichtung zum Bestimmen von rheologischen Eigenschaften einer Schmelze mit mindestens einem Schmelzenkanal, der endseitig mit einer Meßdüse versehen ist, durch die die Schmelze extrudiert wird, mit Mitteln zum Erzeugen und Regeln eines auf die Schmelze wirkenden Gasdrucks und mit Mitteln zum Erfassen bzw. Ermitteln des extrudierten Volumenstromes.

Die rheologischen Eigenschaften und insbesondere die Fließeigenschaften von Polymerschmelzen werden in der Regel mit Hilfe von Rheometern bestimmt. Bei herkömmlichen Rheometern unterliegt die Schmelze einer Druckströmung durch eine Meßdüse vom Typ Schlitzdüse oder Rundlochdüse, wobei Rundlochdüsen auch als Meßkapillaren bezeichnet werden. Man spricht in diesem Falle von Kapillarrheometern. Die bekannten Kapillarrheometer werden entweder durchsatzgesteuert oder spannungsgesteuert betrieben. Bei der durchsatzgesteuerten Fahrweise wird der Volumenstrom durch die Meßdüse vorgegeben. Gemessen wird dann der sich daraufhin einstellende Druckabfall über der Meßdüse. Der Druckabfall bildet hier also die abhängige Meßgröße. Im Gegensatz dazu wird bei der spannungsgesteuerten Fahrweise der Druckabfall über der Meßdüse vorgegeben, indem der dazu notwendige Volumenstrom entsprechend geregelt wird. In diesem Falle bildet der zu regelnde Volumenstrom die Meßgröße.

Bei beiden Versuchsmodi muß der Druckabfall über der Meßdüse überwacht werden, wozu beispielsweise kommerziell erhältliche Druckaufnehmer verwendet werden, die auf der Basis von Dehnungsmeßstreifen arbeiten und eine Genauigkeit von 0,5-1% vom Nennbereich erzielen. Dies führt beispielsweise bei einem Nennbereich von 0-200 bar dazu, daß Drücke unter 2 bar nicht hinreichend reproduzierbar gemessen werden können. Weitere Fehlerquellen bei der Druckmessung mit Hilfe von Dehnungsmeßstreifen ergeben sich aus der Nichtlinearität der Federkennlinie im unteren Nennbereich und daraus, daß die Dehnungsmeßstreifen in direktem Kontakt mit der Schmelze stehen und in der Regel eine ungenügende Stabilität bei Schmelzentemperaturen von über 200 °C aufweisen.

Im allgemeinen setzt man bei der Berechnung der Viskositätsfunktion einer Schmelze aus den gemessenen Druckabfällen und den korrespondierenden Volumenströmen voraus, daß sich beim Durchströmen der Schmelze durch die Meßkapillare ein parabolisches Strömungsprofil ausbildet, wobei die Strömungsgeschwindigkeit an der Wand gegen 0 geht, also Wandhaftung gegeben ist. Nur unter dieser Voraussetzung ist die Bestimmung der Viskositätsfunktion bzw. der Fließkurve nach den Gleichungen (1) und (2) korrekt, wobei hier bewußt auf den Einfluß der Strukturviskosität verzichtet werden soll.

$$\tau = \frac{\Delta p D}{8L} \qquad (1)$$

$$\gamma = \frac{4V}{\pi r^3} \qquad (2)$$

In den Gleichungen (1) und (2) bezeichnet $\tau$ die Schubspannung, $\Delta p$ den Druck im Schmelzenkanal, D den Durchmesser der Meßdüse, L die Länge der Meßdüse, $\gamma$ die Schergeschwindigkeit, V den Volumendurchsatz durch die Meßdüse und r den Radius der Meßdüse.

Es ist hinlänglich bekannt, daß beispielsweise lineares Polyethylen ab einer kritischen Wandschubspannung zu Gleiten beginnt. Ähnliche Phänomene zeigen Polymerschmelzen, die mit Verarbeitungshilfen versehen sind, welche den Fließwiderstand in der Meßdüse reduzieren. Vor Bestimmung der Viskositätsfunktion einer Schmelze mit Hilfe der Gleichungen (1) und (2) muß also zunächst überprüft werden, ob tatsächlich Wandhaftung gegeben ist oder ob ein Gleiten der Schmelze vorliegt. Im letzt genannten Fall sollte dann noch der Betrag der Gleitgeschwindigkeit bestimmt werden, wozu mindestens zwei Experimente erforderlich sind. Der Betrag der Gleitgeschwindigkeit, der über dem Düsendurchmesser konstant ist, bildet den linearen Teil der vorliegenden Blockströmung durch die Meßdüse.

In diesem Zusammenhang ist die voranstehend beschriebene spannungsgesteuerte Fahrweise eines Kapillarrheometers gegenüber der durchsatzgesteuerten Fahrweise von Vorteil. Bei der hinlänglich bekannten Korrektur nach Mooney (1931) wird nämlich die Gleitgeschwindigkeit als Funktion der Schubspannung dadurch bestimmt, daß man Fließkurven, d. h. die Schubspannung als Funktion der Schergeschwindigkeit, mit mindestens zwei Meßdüsen unterschiedlichen Durchmessers ermittelt. Gleiten liegt dann vor, wenn die Fließkurven im Bereich der kritischen Schubspannung an der Wand nicht mehr zur Deckung kommen, die ermittelte Schergeschwindigkeit also vom Düsendurohmesser abhängig ist.

Der vorliegenden Erfindung liegt nun die Aufgabe zugrunde, eine Vorrichtung der eingangs genannten Art - nämlich ein einfaches Rheometer - derartig auszugestalten und weiterzubilden, daß auf einfache Weise nicht nur die Viskositätsfunktion einer Schmelze bestimmt werden kann, sondern auch geprüft werden kann, ob Gleiten vorliegt und - bejahenden Falls die Gleitgeschwindigkeit bestimmt werden kann.

Die erfindungsgemäße Vorrichtung zum Bestimmen von rheologischen Eigenschaften einer Schmelze löst die voranstehende Aufgabe durch die Merkmale des Patentanspruchs 1. Danach ist die eingangs genannte Vorrichtung so ausgestaltet, daß mindestens zwei Schmelzenkanäle vorgesehen sind, daß die Schmelzenkanäle mit Meßdüsen unterschiedlicher Geometrie ausgestattet sind und daß eine Druckverbindung zwischen den Schmelzenkanälen in Form von mindestens einer Verbindungsleitung besteht, so daß in allen Schmelzenkanälen derselbe Gasdruck auf die Schmelze wirkt.

Erfindungsgemäß ist erkannt worden, daß mit Hilfe eines Mehrkanalsystems die für die Bestimmung des Gleitens der Schmelze erforderlichen Experimente auf einfache Weise parallel, d. h. gleichzeitig, durchgeführt werden können. Dies ist nicht nur im Hinblick auf eine zügige Meßführung von Vorteil, sondern erhöht auch die Genauigkeit der Meßergebnisse, wenn - wie mit der erfindungsgemäßen Vorrichtung gewährleistet - in allen Schmelzenkanälen derselbe Druck auf die Schmelze einwirkt.

Da die zu untersuchenden Schmelzen, wie z.B. polymere Werkstoffe, oftmals noch aufgeschmolzen werden müssen und außerdem die Bestimmung der rheologischen Eigenschaften einer Schmelze unter thermisch akzeptablen Bedingungen durchgeführt werden sollten, also beispielsweise bei leicht erhöhter Raumtemperatur, ist es von Vorteil, wenn die Schmelzenkanäle der erfindungsgemäßen Vorrichtung beheizbar sind.

Da die Schmelzenkanäle der erfindungsgemäßen Vorrichtung über mindestens eine Verbindungsleitung miteinander kommunizieren, so daß in allen Schmelzenkanälen derselbe Gasdruck herrscht, ist es ausreichend, wenn lediglich einer der Schmelzenkanäle an eine Gaszuleitung angeschlossen ist, über die das auf die Schmelze wirkende Gas eingeleitet wird. Grundsätzlich kann es sich bei dem den Gasdruck erzeugenden Gas um ein beliebiges Gas handeln; vorteilhafterweise wird hierzu allerdings ein inertes Gas, wie z. B. Stickstoff, eingesetzt, um chemische Reaktionen mit der Schmelze zu vermeiden.

Wie bereits erwähnt, umfaßt die erfindungsgemäße Vorrichtung Mittel zum Erzeugen und Regeln des auf die Schmelze wirkenden Gasdrucks. Dazu könnte mindestens ein Druckaufnehmer zur Messung des in den Schmelzenkanälen herrschenden Gasdrucks vorgesehen sein, dessen Meßwerte einem Regler zugeführt werden, der wiederum in Abhängigkeit von dem Meßwerten des Druckaufnehmers ein Regelventil in der Gaszuleitung ansteuert. Bei dem Regler könnte es sich beispielsweise um einen Proportionalregler handeln.

Um eine gleichförmige Strömung der Schmelze zu gewährleisten, ist in einer vorteilhaften Ausgestaltung der erfindungsgemäßen Vorrichtung jeweils auf dem oberen Meniskus der Schmelze in jedem Schmelzenkanal ein Trennkörper angeordnet, wobei der Trennkörper einen geringfügig kleineren Querschnitt aufweist als der jeweilige Schmelzenkanal. Bei dem Trennkörper könnte es sich beispielsweise um einen Stempel oder eine Kugel handeln.

Der Trennkörper könnte in einer weiteren vorteilhaften Variante der erfindungsgemäßen Vorrichtung nicht nur zum Gewährleisten einer gleichförmigen Strömung der Schmelze dienen, sondern auch zum Erfassen bzw. Ermitteln des extrudierten Volumenstromes. Dazu könnte die erfindungsgemäße Vorrichtung für jeden Schmelzenkanal Mittel zum Erfassen der Position bzw. der Geschwindigkeit des Trennkörpers umfassen, sowie eine Auswerteeinheit vorgesehen sein, der die erfaßten Positions- bzw. Geschwindigkeitsdaten zum Ermitteln des jeweils extrudierten Volumenstromes zugeführt werden. In Anbetracht der in den Schmelzenkanälen der erfindungsgemäßen Vorrichtung herrschenden Druckverhältnisse ist es von Vorteil, wenn die Position des Trennkörpers mit Hilfe mindestens eines druckunempfindlichen Wegaufnehmers erfaßt wird. Dabei könnte es sich beispielsweise um einen induktiven Wegaufnehmer handeln.

Zweckmäßigerweise wird man die Schmelzenkanäle der erfindungsgemäßen Vorrichtung je nach Aufgabenstellung mit unterschiedlichen Meßdüsen ausstatten. Will man beispielsweise die sogenannte Bagley-Korrektur zur Bestimmung des Einlaufdruckverlustes ermitteln, so empfiehlt es sich die Schmelzenkanäle mit Meßkapillaren auszustatten, die einen identischen Durchmesser aber unterschiedliche Längen aufweisen. Soll die sogenannte Mooney-Korrektur zur Erweiterung des Schergeschwindigkeitsbereichs bestimmt werden, so empfiehlt es sich, die Schmelzenkanäle der erfindungsgemäßen Vorrichtung mit Meßkapillaren konstanter Länger aber unterschiedlichen Durchmessers auszustatten.

Besonders vorteilhaft ist es, die Schmelzenkanäle der erfindungsgemäßen Vorrichtung mit Meßkapillaren auszustatten, die ein identisches Längen/Durchmesser-Verhältnis aufweisen aber unterschiedliche Durchmesser aufweisen. In diesem Falle kann nämlich in einem einzigen Experiment ermittelt werden, ob sich die Schergeschwindigkeit bei konstanter Schubspannung ändert, wenn unterschiedliche Düsen zum Einsatz kommen. Dies wird nachfolgend anhand eines Ausführungsbeispiels näher erläutert werden.

Schließlich sei noch auf die Möglichkeit hingewiesen, in den Verbindungsleitungen zwischen den Schmelzenkanälen jeweils ein Absperrventil anzuordnen, so daß die erfindungsgemäße Vorrichtung beispielsweise auch als Einkanalsystem betrieben werden kann und einfach um weitere Schmelzenkanalmodule erweitert werden kann.

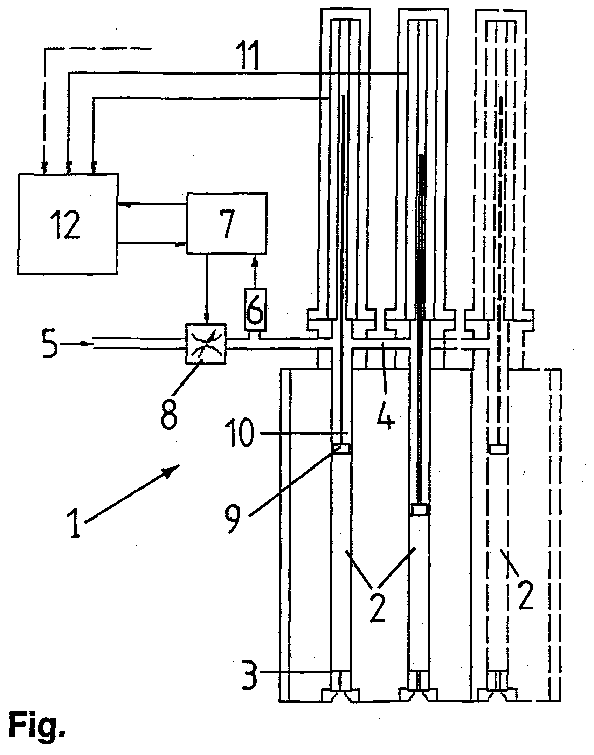

Es gibt nun verschiedene Möglichkeiten, die Lehre der vorliegenden Erfindung in vorteilhafterweise auszugestalten und weiterzubilden. Dazu ist einerseits auf die dem Patentanspruch 1 nachgeordneten Ansprüche, andererseits auf die nachfolgende Erläuterung eines Ausführungsbeispiels der Erfindung anhand der Zeichnung zu verweisen.

In der Zeichnung zeigt die einzige Figur schematisch den konstruktiven Aufbau einer erfindungsgemäßen Vorrichtung zum Bestimmen von rheologischen Eigenschaften einer Schmelze.

Die hier dargestellte Vorrichtung 1 umfaßt drei Schmelzenkanäle 2, von denen einer lediglich gestrichelt dargestellt ist, um anzudeuten, daß die gesamte Vorrichtung 1 modular aufgebaut ist, also um weitere Schmelzenkanäle 2 erweiterbar ist. Jeder der Schmelzenkanäle 2 ist endseitig mit einer Meßdüse 3 versehen, durch die die Schmelze extrudiert wird. Des weiteren sind noch Mittel zum Erzeugen und Regeln eines auf die Schmelze wirkenden Gasdrucks und Mittel zum Erfassen bzw. Ermitteln des extrudierten Volumenstromes vorhanden, die nachfolgend näher erläutert werden.

Erfindungsgemäß sind mindestens zwei Schmelzenkanäle 2 vorgesehen, die mit Meßdüsen 3 unterschiedlicher Geometrie ausgestattet sind. Außerdem besteht eine Druckverbindung zwischen den Schmelzenkanälen 2 in Form einer Verbindungsleitung 4, so daß in allen Schmelzenkanälen 2 derselbe Gasdruck auf die Schmelze wirkt.

Die Schmelzenkanäle 2 sind beheizbar, so daß sie im hier dargestellten Ausführungsbeispiel nicht nur der Aufnahme sondern auch der Temperierung der Schmelze dienen. Die hier beschriebene Vorrichtung 1 ermöglicht auch beispielsweise ein Aufschmelzen von polymeren Werkstoffen in den Schmelzenkanälen 2.

Im hier dargestellten Ausführungsbeispiel wird der auf die Schmelze wirkende Gasdruck mit Hilfe eines Gases, wie z.B. Stickstoff erzeugt, das über eine Gaszuleitung 5 in die Schmelzenkanäle 2 eingeleitet wird. Mit Hilfe eines Druckaufnehmers 6, der hier an die Gaszuleitung 5 angeschlossen ist, wird der auch in den Schmelzenkanälen 2 herrschende Gasdruck unter thermisch akzeptablen Bedingungen, bei leicht erhöhter Raumtemperatur gemessen. Im hier dargestellten Ausführungsbeispiel handelt es sich bei dem Druckaufnehmer 4 um einen Präzisionsdruckaufnehmer, der eine Genauigkeit von < 0,05% vom Nennwert aufweist. Die Meßwerte des Druckaufnehmers 6 werden einem Regler 7, hier einem Proportionalregler, zugeführt, der wiederum in Abhängigkeit von den Meßwerten des Druckaufnehmers 6 ein Regelventil 8 in der Gaszuleitung 5 ansteuert.

In jedem der Schmelzenkanäle 2 befindet sich auf der unter Druck befindlichen Schmelzenoberfläche, dem oberen Meniskus der Schmelze, ein Trennkörper - im hier dargestellten Ausführungsbeispiel ein zylinderförmiger Stempel 9, der einen geringfügig kleineren Querschnitt aufweist als der jeweilige Schmelzenkanal 2. Jeder Stempel 9 ist hier mit einem Eisenanker 10 verbunden. Außerdem sind für jeden Schmelzenkanal 2 Mittel zum Erfassen der Position bzw. der Geschwindigkeit des Stempels 9 vorgesehen, die im hier dargestellten Ausführungsbeispiel in Form von druckunempfindlichen, induktiven Wegaufnehmern 11 realisiert sind, die die Wegänderung der Eisenanker 10 messen. Die Meßdaten der Wegaufnehmer 11 werden einer Auswerteeinheit 12 zugeleitet, die aus den bekannten Geometrien der Schmelzenkanäle 2 und den erfaßten Positions- bzw. Geschwindigkeitsdaten den jeweils extrudierten Volumenstrom ermittelt, um damit die korrespondierenden Schergeschwindigkeiten zu ermitteln.

Wie bereits erwähnt, ist jeder Schmelzenkanal 2 der erfindungsgemäßen Vorrichtung endseitig mit einer Meßdüse 3 - im hier dargestellten Ausführungsbeispiel einer Meßkapillare - versehen, durch welche die Schmelze extrudiert wird. Entgegen der Darstellung in der einzigen Figur soll nachfolgend der Fall näher erläutert werden, daß Meßkapillaren mit gleichem Längen/Durchmesser-Verhältnis zum Einsatz kommen, die jedoch unterschiedliche Durchmesser aufweisen sollen. Die Schmelze wird in einem zu spezifizierenden Spannungsbereich extrudiert. Dazu wird den Schmelzenkanälen beispielsweise Stickstoff in einem Druckbereich von bis zu 300 bar zugeführt. Der zwischen der Stickstoffquelle und den Schmelzenkanälen befindliche Regelkreis sollte minimale Drücke von 0,1 bar regeln können, um zu gewährleisten, daß in den Schmelzenkanälen immer eine konstante Schubspannung $\tau$ nach Gleichung (1)

$$\tau = \frac{\Delta p D}{8L} \qquad\qquad (1)$$

an der Wand der Schmelzenkanäle vorliegt, obgleich die Schmelzenkanäle mit Meßkapillaren ausgestattet sind, die aufgrund ihrer Geometrie unterschiedliche Fließwiderstände verursachen. So stellen sich aufgrund der unterschiedlichen Düsengeometrien bei konstant zu haltendem Druck $\Delta p$ in den Schmelzenkanälen unterschiedliche Volumendurchsätze V ein. Die entsprechende scheinbare Schergeschwindigkeit $\gamma$ an der Wand kann nun nach Gleichung (2) bestimmt werden als

$$\gamma = \frac{4V}{\pi r^3} \qquad\qquad (2)$$

wobei r der Radius der Meßkapillaren ist.

Bei einer wie voranstehend beschriebenen Versuchsanordnung kann nun im Rahmen eines einzigen Experiments ermittelt werden, ob sich die Schergeschwindigkeit bei konstanter Schubspannung ändert, wenn Meßkapillaren unterschiedlicher Geometrie zum Einsatz kommen. Liegen die Fließkurven deckungsgleich übereinander, so hat der Radius der Meßkapillaren keinen Einfluß auf die Fließkurve. Daraus kann geschlossen werden, daß Wandhaftung der Schmelze vorliegt. Ergeben sich aber bei konstanter Schubspannung unterschiedliche Schergeschwindigkeiten, so muß man von einem Gleiten der Schmelze an der Wand ausgehen. Gemäß der Methode von Mooney läßt sich der Betrag der Gleitgeschwindigkeit dann direkt berechnen.

Experiment und Berechnungsmethode sind mit herkömmlichen Kapillarrheometern nur sehr zeitaufwendig zu realisieren, da in einem Kapillarrheometer mit nur einem Schmelzenkanal mindestens zwei Experimente mit unterschiedlichen Düsendurchmessern aber konstantem Längen/Durchmesser-Verhältnis durchgeführt werden müssen. Das voranstehend beschriebene Mehrkanalsystem führt die üblichen zeitaufwendigen sequentiellen Experimente parallel, also gleichzeitig, durch. Wichtige Voraussetzung ist dabei, daß in allen Schmelzenkanälen ein konstant zu haltender Druck gewährleistet ist.

**Patentansprüche**

1. Vorrichtung zum Bestimmen von rheologischen Eigenschaften einer Schmelze

   - mit mindestens einem Schmelzenkanal (2), der endseitig mit einer Meßdüse (3) versehen ist, durch die die Schmelze extrudiert wird,
   - mit Mitteln zum Erzeugen und Regeln eines auf die Schmelze wirkenden Gasdrucks und
   - mit Mitteln zum Erfassen bzw. Ermitteln des extrudierten Volumenstromes,

   **dadurch gekennzeichnet**, daß mindestens zwei Schmelzenkanäle (2) vorgesehen sind, daß die Schmelzenkanäle (2) mit Meßdüsen (3) unterschiedlicher Geometrie ausgestattet sind und daß eine Druckverbindung zwischen den Schmelzenkanälen (2) in Form von mindestens einer Verbindungsleitung (4) besteht, so daß in allen Schmelzenkanälen (2) derselbe Gasdruck auf die Schmelze wirkt.

2. Vorrichtung nach Anspruch 1, dadurch gekennzeichnet, daß die Schmelzenkanäle (2) beheizbar sind.

3. Vorrichtung nach einem der Ansprüche 1 oder 2, dadurch gekennzeichnet, daß an einen der Schmelzenkanäle (2) eine Gaszuleitung (5) für ein vorzugsweise inertes Gas angeschlossen ist, über das der auf die Schmelze wirkende Gasdruck erzeugt wird.

4. Vorrichtung nach Anspruch 3, dadurch gekennzeichnet, daß der Gasdruck mit Hilfe von Stickstoff erzeugt wird.

5. Vorrichtung nach einem der Ansprüche 3 oder 4, dadurch gekennzeichnet, daß mindestens ein Druckaufnehmer (6) zur Messung des in den Schmelzenkanälen (2) herrschenden Gasdrucks vorgesehen ist und daß ein Regler (7) vorgesehen ist, dem die Meßwerte des Druckaufnehmers (6) zugeführt werden und der in Abhängigkeit von den Meßwerten des Druckaufnehmers (6) ein Regelventil (8) in der Gaszuleitung (5) ansteuert.

6. Vorrichtung nach Anspruch 5, dadurch gekennzeichnet, daß es sich bei dem Regler (7) um einen Proportionalregler handelt.

7. Vorrichtung nach einem der Ansprüche 1 bis 6, dadurch gekennzeichnet, daß auf dem oberen Meniskus der Schmelze in jedem Schmelzenkanal (2) ein Trennkörper (9) angeordnet ist, wobei der Trennkörper (9) einen geringfügig kleineren Querschnitt aufweist als der jeweilige Schmelzenkanal (2).

8. Vorrichtung nach Anspruch 7, dadurch gekennzeichnet, daß für jeden Schmelzenkanal (2) Mittel zum Erfassen der Position bzw. der Geschwindigkeit (11) des Trennkörpers (9) vorgesehen sind und daß eine Auswerteeinheit (12) vorgesehen ist, der die erfaßten Positions- bzw. Geschwindigkeitsdaten zum Ermitteln des jeweils extrudierten Volumenstroms zugeführt werden.

9. Vorrichtung nach Anspruch 8, dadurch gekennzeichnet, daß die Position des Trennkörpers (9) mit Hilfe mindestens eines druckunempfindlichen Wegaufnehmers (11) erfaßt wird.

10. Vorrichtung nach Anspruch 8, dadurch gekennzeichnet, daß die Position des Trennkörpers (9) mit Hilfe mindestens eines induktiven Wegaufnehmers (11) erfaßt wird.

11. Vorrichtung nach einem der Ansprüche 7 bis 10, dadurch gekennzeichnet, daß es sich bei dem Trennkörper um einen Stempel (9) oder eine Kugel handelt.

12. Vorrichtung nach einem der Ansprüche 1 bis 11, dadurch gekennzeichnet, daß die Schmelzenkanäle mit Meßkapillaren ausgestattet sind und daß die Meßkapillaren identische Durchmesser und unterschiedliche Längen aufweisen.

13. Vorrichtung nach einem der Ansprüche 1 bis 11, dadurch gekennzeichnet, daß die Schmelzenkanäle mit Meßkapillaren ausgestattet sind und daß die Meßkapillaren unterschiedliche Durchmesser und identische Längen aufweisen.

14. Vorrichtung nach einem der Ansprüche 1 bis 11, dadurch gekennzeichnet, daß die Schmelzenkanäle mit Meßkapillaren ausgestattet sind, die ein identisches Längen/Durchmesser-Verhältnis aufweisen aber unterschiedliche Durchmesser aufweisen.

15. Vorrichtung nach einem der Ansprüche 1 bis 14, dadurch gekennzeichnet, daß in der Verbindungsleitung zwischen den Schmelzenkanälen ein Absperrventil angeordnet ist.

**Fig.**

**Europäisches Patentamt**

**EUROPÄISCHER RECHERCHENBERICHT**

Nummer der Anmeldung

EP 99 12 2442

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 4 723 442 A (MANNING R E ET AL) 9. Februar 1988 (1988-02-09) | 1-6,15 | G01N11/08 |
| Y | * Spalte 2, Zeilen 10-28; Spalte 2, Zeile 52 - Spalte 5, Zeile 2; Spalte 6, Zeile 18 - Spalte 7, Zeile 55; Abbildung 1 * | 7-14 | |
| Y | JP 58 088637 A (NIPPON GOSEI GOMU KK) 26. Mai 1983 (1983-05-26) | 7-12 | |
| A | * Abbildungen 3,4 * -& PATENT ABSTRACTS OF JAPAN vol. 7, no. 188 (P-217), 17. August 1983 (1983-08-17) & JP 58 088637 A (...) * Zusammenfassung * | 1,2,5 | |
| Y | WO 97 10492 A (GOETTFERT WERKSTOFF PRÜFMASCHINEN) 20. März 1997 (1997-03-20) | 12-14 | |
| A | * Seite 13, Absatz 3 - Seite 16, Absatz 6; Abbildungen 1,4 * | 1,5 | |
| A | US 4 425 790 A (BICE W L ET AL) 17. Januar 1984 (1984-01-17) * Spalte 1, Zeile 27 - Spalte 4, Zeile 51; Abbildung 1 * | 1,2,5, 12-14 | RECHERCHIERTE SACHGEBIETE (Int.Cl.7)<br><br>G01N |
| A | US T960004 I (NEVILLE G E) 5. Juli 1977 (1977-07-05) * Zusammenfassung; Abbildung * | 1,2,5,12 | |
| A | DE 42 20 157 A (BÜHLER AG) 21. Januar 1993 (1993-01-21) * Spalte 1, Zeile 64 - Spalte 3, Zeile 19; Spalte 3, Zeile 53 - Spalte 4, Zeile 11; Abbildung 3 * | 1,5,14 | |

Der vorliegende Recherchenbericht wurde für alle Patentansprüche erstellt

| Recherchenort | Abschlußdatum der Recherche | Prüfer |
|---|---|---|
| BERLIN | 6. April 2000 | Johnson, K |

## ANHANG ZUM EUROPÄISCHEN RECHERCHENBERICHT
## ÜBER DIE EUROPÄISCHE PATENTANMELDUNG NR.

EP 99 12 2442

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-04-2000

| Im Recherchenbericht angeführtes Patentdokument | Datum der Veröffentlichung | Mitglied(er) der Patentfamilie | Datum der Veröffentlichung |
|---|---|---|---|
| US 4723442 A | 09-02-1988 | US 4658636 A | 21-04-1987 |
| JP 58088637 A | 26-05-1983 | KEINE | |
| WO 9710492 A | 20-03-1997 | EP 0850403 A<br>US 5959195 A | 01-07-1998<br>28-09-1999 |
| US 4425790 A | 17-01-1984 | KEINE | |
| US T960004 I | 05-07-1977 | KEINE | |
| DE 4220157 A | 21-01-1993 | CH 682348 A | 31-08-1993 |

EPO FORM P0461

Für nähere Einzelheiten zu diesem Anhang : siehe Amtsblatt des Europäischen Patentamts, Nr.12/82